# EUROPEAN PATENT SPECIFICATION

(11) **EP 4 181 808 B1**
(45) Date of publication and mention of the grant of the patent: **20.05.2026**
(21) Application number: 21755154.8
(22) Date of filing: 16.07.2021
(51) Int. Cl.: A61B 18/14

(54) **CATHETER FOR FOCAL CARDIAC ABLATION BY IRREVERSIBLE ELECTROPORATION**
KATHETER ZUR FOKALEN KARDIALEN ABLATION DURCH IRREVERSIBLE ELEKTROPORATION
CATHÉTER D'ABLATION CARDIAQUE FOCALISÉE PAR ÉLECTROPORATION IRRÉVERSIBLE

(30) Priority: 16.07.2020 US 202063052823 P
(43) Date of publication of application: 24.05.2023
(73) Proprietor: Boston Scientific Scimed Inc., Maple Grove, Minnesota 55311 (US)
(72) Inventor: KOOP, Brendan, E., Ham Lake, Minnesota 55304 (US); DE KOCK, Andrew, L., Ham Lake, Minnesota 55304 (US); SHUROS, Allan, C., St. Paul, Minnesota 55116 (US); MAIERHOFER, Edward, J., Brooklyn Park, Minnesota 55443 (US)
(74) Representative: Pfenning, Meinig & Partner mbB
(86) International application number: PCT/US2021/042021
(87) International publication number: WO 2022/016081

(56) References cited:
- EP-A1- 3 173 123
- US-A1- 2020 129 230
- US-B2- 7 416 549

## Description

### TECHNICAL FIELD

The present invention, which is defined in claims 1 and 10, relates to catheters for the ablation of cardiac tissue. More specifically, the invention relates to catheters for focal ablation of cardiac tissue by irreversible electroporation. Any methods disclosed hereinafter do not form part of the scope of the invention, and are mentioned for illustrative purposes only.

### BACKGROUND

Aberrant conductive pathways can disrupt the normal path of the heart's electrical impulses. The aberrant conductive pathways can create abnormal, irregular, and sometimes life-threatening heart rhythms called arrhythmias. Ablation of cardiac tissue is one way of treating arrhythmias and restoring normal conduction. The specific cardiac tissue can be located or mapped using mapping electrodes of a mapping catheter. After mapping, the physician may ablate the aberrant tissue.

Precision, point-by-point, or focal, cardiac ablation is generally accomplished using radio frequency (RF) energy. In radio frequency (RF) ablation, RF energy is directed from an ablation electrode through tissue to an electrode to ablate the tissue and form a lesion. RF energy destroys tissue indiscriminately through cell necrosis, which can result to damage to untargeted tissue, such as nerve and arterial tissues, for example. RF ablation can create other undesired effects, such as tissue charring and steam pops due to the heat generated by the RF energy.

### BACKGROUND ART

Document US 2020/129230 A1 describes a system for ablation, including an inner electrode assembly with an inner elongate shaft and a distal electrode, an outer electrode assembly having an outer elongate shaft and a proximal electrode, wherein the outer electrode assembly defines a central passage configured to slidably receive the inner electrode assembly, wherein the distal electrode or the proximal electrode is axially moveable with respect to the other, and an energy source configured to be electrically connected to the distal electrode and the proximal electrode and configured to deliver a pulsed electric field (PEF). EP3173123A1 provides methods and apparatus for pulsed electric field ("PEF") neuromodulation via an intra-to-extravascular ("ITEV") approach, e.g., to effectuate irreversible electroporation or electrofusion, necrosis and/or inducement of apoptosis, alteration of gene expression, changes in cytokine upregulation, and other conditions in target neural fibers.

### SUMMARY OF THE INVENTION

The invention is defined by the claims. It relates to a catheter for focal cardiac ablation by irreversible electroporation as defined in independent claim 1 or 10. Advantageous embodiments can be found in the dependent claims, and in the following description and the appended figures. The following Examples form part of the disclosure and may be helpful for understanding the invention.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIGS. 1A and 1B are schematic perspective views of a distal end of a catheter for focal cardiac ablation by irreversible electroporation in undeployed and deployed configurations, respectively, according to some embodiments of this disclosure.
FIG. 2 is a schematic side view of the distal end of a catheter for focal cardiac ablation by irreversible electroporation deployed into tissue, according to some embodiments of this disclosure.
FIG. 3 is a schematic side view of the distal end of another catheter for focal cardiac ablation by irreversible electroporation deployed into tissue, according to some embodiments of this disclosure.
FIG. 4 is a schematic side view of the distal end of yet another catheter for focal cardiac ablation by irreversible electroporation deployed into tissue, according to some embodiments of this disclosure.
FIGS. 5A and 5B are schematic perspective views of a distal end of a catheter for focal cardiac ablation by irreversible electroporation in undeployed and deployed configurations, respectively, according to some other embodiments of this disclosure.
FIGS. 6A and 6B are schematic perspective views of a distal end of a catheter for focal cardiac ablation by irreversible electroporation in undeployed and deployed configurations, respectively, according to some other embodiments of this disclosure.

While the disclosure is amenable to various modifications and alternative forms, specific embodiments have been shown by way of example in the drawings and are described in detail below. The intention, however, is not to limit the invention to the particular embodiments described. On the contrary, the disclosure is intended to cover all modifications, equivalents, and alternatives falling within the scope of the invention as defined by the appended claims.

### DETAILED DESCRIPTION

irreversible electroporation uses trains of short, high voltage pulses to kill cells by forming lethal nanopores in the cell membranes. The damaged cells then die through apoptosis. The voltage pulses of irreversible electroporation can be targeted to kill myocardium, and leave other tissues relatively unscathed, thus avoiding the undesired side effects of RF ablation. However, forming transmural lesions in thick myocardium can be difficult with irreversible electroporation. In addition, ablation of heart tissue poses a challenge in that the heart is constantly moving during an ablation procedure. As a result, it can be difficult to maintain stable contact between an ablating electrode and the target tissue. Embodiments of the present disclosure provide catheters and methods for focal cardiac ablation by irreversible electroporation that reduce these problems.

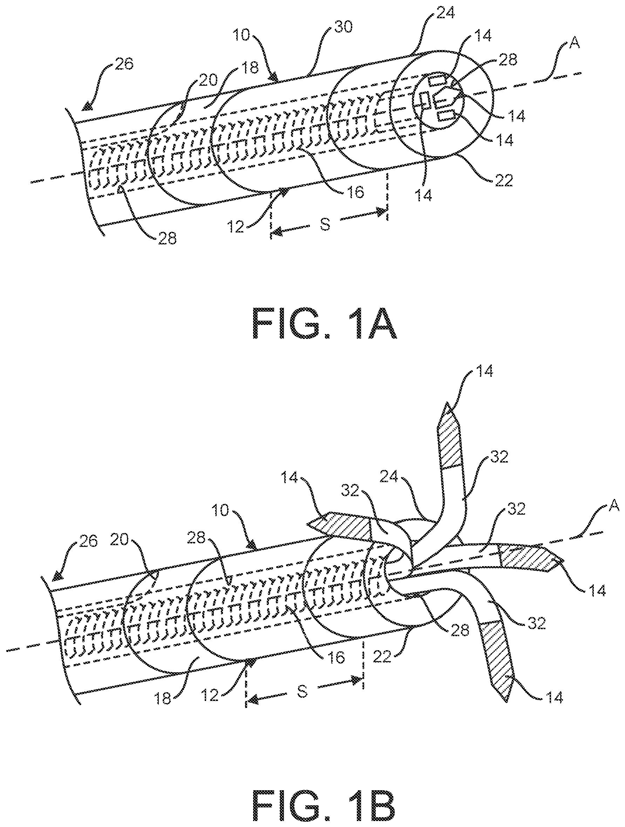

FIGS. 1A and 1B are schematic perspective views of a distal end of a catheter for focal cardiac ablation by irreversible electroporation, according to some embodiments of this disclosure. FIG. 1A shows a catheter 10 including flexible catheter body 12, a plurality of tines 14 (four shown), a flexible shaft 16, a return electrode 18, and an electrical conductor 20. In some embodiments, the catheter 10 may further include an atraumatic tip 22, as shown in FIG. 1A. The catheter body 12 extends from a proximal end 26 to a distal end 24. The catheter body 12 forms a lumen 28 extending from the proximal end 26 to the distal end 24. The catheter body 12 defines a longitudinal axis A. The catheter body 12 may be formed of a flexible, insulative, biocompatible material known in the art, such as polyisobutylene polyurethane, silicone or polyether block amide, for example. The catheter body 12 may further include a metallic braiding.

The flexible shaft 16 includes an electrically conductive biocompatible material, such as a platinum iridium alloy, gold, stainless-steel, a titanium alloy, or a nickel-cobalt alloy, such as MP35N, for example. The flexible shaft 16 may further include an insulating polymer coating. In some embodiments, the flexible shaft 16 is in the form of a wire coil. The electrical conductor 20 is also formed of any of the previously mentioned electrically conductive materials and may also further include an insulating polymer coating.

The optional atraumatic tip 22 may formed of a biocompatible material, such as polyether ether ketone (PEEK), polyisobutylene polyurethane, silicone, polyether block amide or titanium, for example. The atraumatic tip 22 is disposed at the distal end 24 and forms the tip of the catheter 10 when the catheter 10 is in the undeployed configuration, as shown in FIG. 1A.

The plurality of tines 14 is disposed at the distal end 24 of the catheter body 12. The flexible shaft 16 is mechanically and electrically coupled to the plurality of tines 14 and extends through the lumen 28 from the proximal end 26 of the catheter body 12. FIG. 1A shows the plurality of tines 14 in an undeployed configuration, with each tine of the plurality of tines 14 substantially contained within the lumen 28.

The return electrode 18 is disposed on an outer surface 30 of the catheter body 12. In the embodiment shown in FIG. 1A, the return electrode 18 includes a ring extending around a circumference of the catheter body 12.

The return electrode 18 is spaced apart from the distal end 24 of the catheter body 12 by a distance S, which may be as little as 1 mm, 2 mm, 3 mm, 4 mm or 5 mm, or as much as 6 mm, 7 mm, 8 mm, 9 mm or 10 mm, or within any range defined between any two of the foregoing values, such as 1 mm to 10 mm, 2 mm to 9 mm, 3 mm to 8 mm, 4 mm to 7 mm, 5 mm to 6 mm, 1 mm to 5 mm, 6 mm to 10 mm, 2 mm to 5 mm, 3 mm to 4 mm or 2 mm to 6 mm, for example.

The electrical conductor 20 is electrically coupled to the return electrode 18. The electrical conductor 20 extends through the catheter body 12 from the proximal end 26. In some embodiments, the electrical conductor 20 extends through the lumen 28 from the proximal end 26 of the catheter body 12, as shown in FIG. 1A. In some other embodiments, the electrical conductor 20 may extend from the proximal end 26 through a separate lumen (not shown) formed by the catheter body 12. In some other embodiments, the electrical conductor 20 may molded or formed into the catheter body 12.

The flexible shaft 16 may be moved distally to deploy the plurality of tines 14 from the lumen 28, as shown in FIG. 1B. The plurality of tines 14 are formed of an electrically conductive material having a shape memory, for example Nitinol or a gold/stainless steel alloy, such that each tine of the plurality of tines 14 can biased to be curved configuration when unrestrained and outside of the lumen 28 as shown in FIG. 1B, and can be in a linear configuration when restrained within the lumen 28, as shown in FIG. 1A. Each tine of the plurality of tines 14 can be pointed for ease in entering tissue. In the curved configuration shown in FIG. 1B, each tine of the plurality of tines 14 forms a curve extending from the distal end 24 of the catheter body 12 and away from the longitudinal axis A.

As shown in FIG. 1B, each tine of the plurality of tines 14 may further include an insulating layer 32. The insulating layer 32 may be useful to prevent applying a voltage to the atraumatic tip 22 if the atraumatic tip 22 is formed of a conductive material. The insulating layer 32 may also be useful in some embodiments in which the flexible shaft 16 is in the form of a multifilar coil with each filar electrically connected to a different tine of the plurality of tines 14 so that they may be used in mapping the heart prior to the focal cardiac ablation. During such mapping, the plurality of tines 14 are deployed without penetrating tissue.

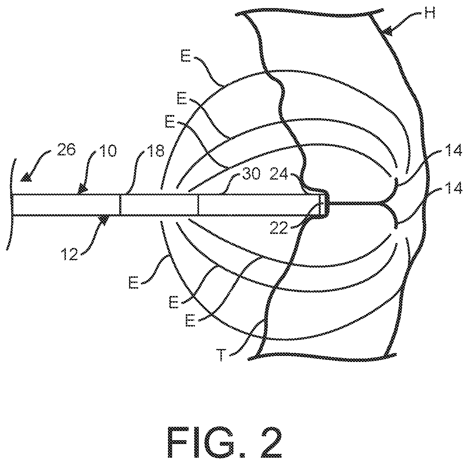

FIG. 2 is a schematic side view of the distal end 24 of the catheter 10 deployed into tissue, according to some embodiments of this disclosure. Considering FIGS. 1A, 1B and 2 together, in use, the distal end 24 of the catheter body 12 of the catheter 10 is inserted into a heart H and adjacent to tissue T containing cells to be ablated. The flexible shaft 16 (FIGS. 1A and 1B) is moved toward the distal end 24 to deploy plurality of tines 14 from the lumen 28 and into the tissue T. As each tine of the plurality of tines 14 moves out of the lumen 28 they transition from the liner configuration of FIG. 1A to the curved configuration shown in FIG. 1B. Each tine of the plurality of tines 14 penetrates the tissue T and, unconstrained by the catheter body 12, self-biases to the curved configuration outside of the lumen 28 as it penetrates through the tissue T, carving a curved path through the tissue T. So deployed, the plurality of tines 14 stabilizes the catheter 10 at the desired location before, during and after ablation. Once deployed, a series of voltage pulses is applied between the plurality of tines 14 and the return electrode 18 to form an electric field E of sufficient strength to cause irreversible electroporation of the cells of the tissue T to be ablated. Various sizes and deployment depths of the plurality of tines 14 can be used to target specific portions of the tissue T. The distal end 24 of the catheter body 12 does not penetrate into the tissue T.

Each tine of the plurality of tines 14 may penetrate tissue T to a depth of as little as 1 mm, 1.5 mm, 2 mm, 2.5 mm, 3 mm or 3.5 mm or as great as 4 mm, 4.5 mm, 5 mm, 5.5 mm, 6 mm, 6.5 mm or 7 mm, or within any range defined between any two of the foregoing values, such as 1 mm to 7 mm, 1.5 mm to 6.5 mm, 2 mm to 6 mm, 2.5 mm to 5.5 mm, 3 mm to 5 mm, 4 mm to 4.5 mm, 1 mm to 2 mm, 2 mm to 7 mm or 1 mm to 3 mm, for example.

In some embodiments, each tine of the plurality of tines 14 may have a rectangular cross-section having a cross-sectional length and a cross-sectional width. The cross-sectional length may be as small 0.25 mm, 0.30 mm, 0.35 mm, 0.40 mm, 0.45 mm or 0.50 mm, or as large as 0.55 mm, 0.60 mm, 0.65 mm, 0.70 mm, 0.75 mm or 0.80 mm, or within any range defined between any two of the foregoing values, such as 0.25 mm to 0.80 mm, 0.30 mm to 0.75 mm, 0.35 mm to 0.70 mm, 0.40 mm to 0.65 mm, 0.45 mm to 0.60 mm, 0.50 mm to 0.55 mm, 0.30 mm to 0.60 mm, 0.25 mm to 0.50 mm, or 0.55 mm to 0.75 mm, for example. The cross-sectional width may be as small 0.10 mm, 0.13 mm, 0.15 mm, 0.18 mm, 0.20 mm or 0.23 mm, or as large as 0.25 mm, 0.28 mm, 0.30 mm, 0.33 mm, 0.36 mm or 0.38 mm, or within any range defined between any two of the foregoing values, such as 0.10 mm to 0.38 mm, 0.13 mm to 0.36 mm, 0.15 mm to 0.33 mm, 0.18 mm to 0.30 mm, 0.20 mm to 0.28 mm, 0.23 mm to 0.25 mm, 0.20 mm to 0.30 mm, 0.10 mm to 0.20 mm, or 0.25 mm to 0.38 mm, for example.

The electric field E may be as low as 300 V/cm, 350 V/cm, 400 V/cm, 450 V/cm, 500 V/cm, 550 V/cm or 600 V/cm, or as high as 650 V/cm, 700 V/cm, 750 V/cm, 800 V/cm, 850 V/cm, 900 V/cm, 950 V/cm or 1,000 V/cm or within any range defined between any two of the foregoing values, such as 300 V/cm to 1,000 V/cm, 350 V/cm to 950 V/cm, 400 V/cm to 900 V/cm, 450 V/cm to 850 V/cm, 500 V/cm to 800 V/cm, 550 V/cm to 750 V/cm, 600 V/cm to 700 V/cm, 300 V/cm to 650 V/cm, 400 V/cm to 500 V/cm, or 700 V/cm to 900 V/cm, for example.

It is believed that the electric field E can be concentrated further into the depth of the tissue T to form transmural lesions in thick myocardium by deploying the plurality of tines 14 deep within the tissue T and generating the electric field between the plurality of tines 14 and the return electrode 18 disposed on the outer surface 30 of the catheter body 12 and spaced apart from the distal end 24.

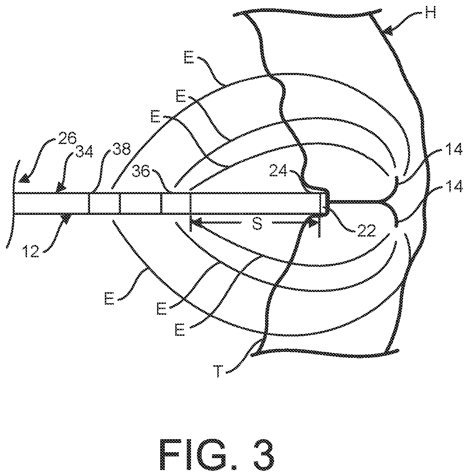

FIG. 3 is a schematic side view of the distal end 24 of another catheter for focal cardiac ablation by irreversible electroporation deployed into tissue, according to some embodiments of this disclosure. FIG. 3 shows a catheter 34. The catheter 34 is substantially identical to the catheter 10 described above, except that the single ring of the return electrode 18 as shown the embodiments of FIGS. 1A, 1B and 2, is replaced with a first ring 36 and a second ring 38, each ring extending around the circumference of the catheter body 12, the second ring 38 disposed proximally from the first ring 36. In some embodiments, the electrical conductor 20 (FIGS. 1A and 1B) is electrically connected to both the first ring 36 and the second ring 38. In this way, the electric field E may be shaped for more effective ablation of the tissue T. In some other embodiments, the electrical conductor 20 (FIGS. 1A and 1B) includes two separate conductors (not shown), electrically isolated from each other and each conductor electrically connected to the first ring 36 or the second ring 38. In this way, the strength and size of the electrical field E may be tuned to provide more targeted ablation of the tissue T. While the return electrode of the embodiment shown in FIG. 3 includes two rings (the first ring 36 and the second ring 38), it is understood that the disclosure includes embodiments having a return electrode including more than two rings.

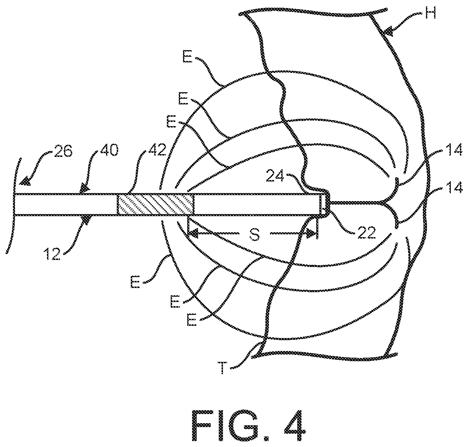

FIG. 4 is a schematic side view of the distal end 24 of yet another catheter for focal cardiac ablation by irreversible electroporation deployed into tissue, according to some embodiments of this disclosure. FIG. 4 shows a catheter 40. The catheter 40 is substantially identical to the catheter 10 described above, except that the single ring of the return electrode 18 as shown the embodiments of FIGS. 1A, 1B and 2, is replaced with a wire coil 42 extending around the circumference of the catheter body 12. The wire coil 42 may be more flexible than the single ring of the return electrode of FIGS 1A, 1B and 2 described above, and may thus be longer without significantly decreasing the flexibility of the catheter body 12. The longer return electrode provided by the wire coil 42 may shape the electric field E for more effective ablation of the tissue T. While in some embodiments, the the wire coil 42 is in the form of a coil, in some other embodiments, the wire coil 42 is in the form of a mesh or a hypo tube cut in a flexible pattern.

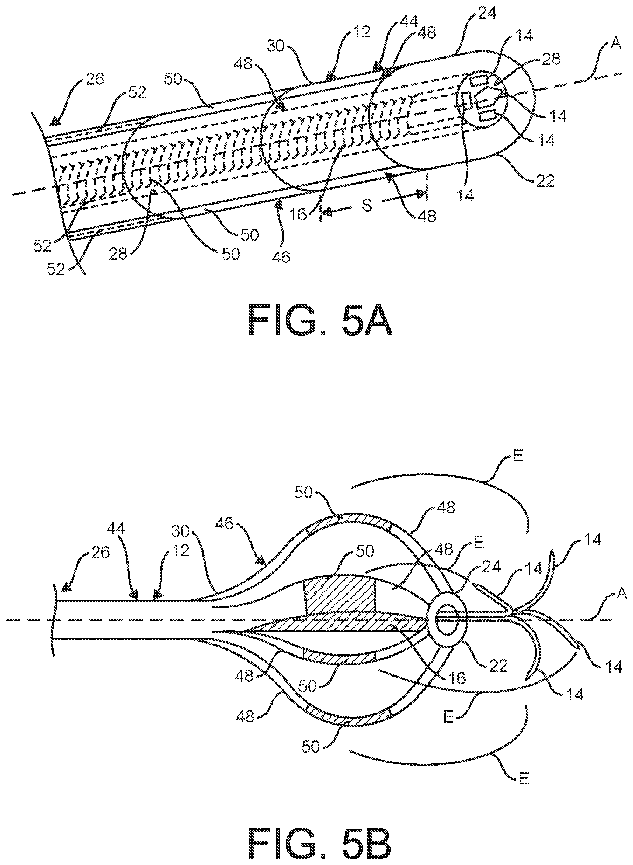

FIGS. 5A and 5B are schematic perspective views of a distal end 24 of a catheter for focal cardiac ablation by irreversible electroporation in undeployed and deployed configurations, respectively, according to some other embodiments of this disclosure. FIG. 5A shows a catheter 44 in the undeployed configuration. The catheter 44 is substantially identical to the catheter 10 described above, except that the catheter body 12 further includes a deployable array 46 including a plurality of splines 48, the return electrode 18 is replaced with a plurality of return electrodes 50, and the electrical conductor 20 is replaced with a plurality of electrical conductors 52. Each spline of the plurality of splines 48 includes at least one return electrode of the plurality of electrodes 50. Each return electrode of the plurality of return electrodes 50 is electrically coupled to at least one of electrical conductors 52 of the plurality of electrical conductors 52. Each electrical conductor of the plurality of electrical conductors 52 may be substantially identical to the electrical conductor 20 described above. Although the catheter 44 includes four splines 48 (see FIG. 5B), only three are visible in FIG. 5A.

In some embodiments, the plurality of splines 48 are formed of the same material making up the rest of the catheter body 12, such polyether block amide, polyisobutylene polyurethane or silicone. In some embodiments, the plurality of splines 48 may further include a material having a shape memory, for example Nitinol or a gold/stainless steel alloy.

The plurality of splines 48 are configured to transition between an undeployed configuration shown in FIG. 5A, to a deployed configuration in FIG. 5B. The heart H and tissue T are omitted for clarity of illustration. As shown in FIG. 5A, in the undeployed configuration, each spline of the plurality of splines 48 is substantially parallel to the longitudinal axis A. As shown in FIG. 5B, in the deployed configuration, each spline of the plurality of splines 48 is bowed radially outward from the longitudinal axis. Deploying the deployable array 46 may occur before, after or coincidental with deploying the plurality of tines 14, as describe above in reference to FIG. 2. In this way, the strength and size of the electrical field E may be tuned and the electrical field # steered to provide more targeted ablation of the tissue T.

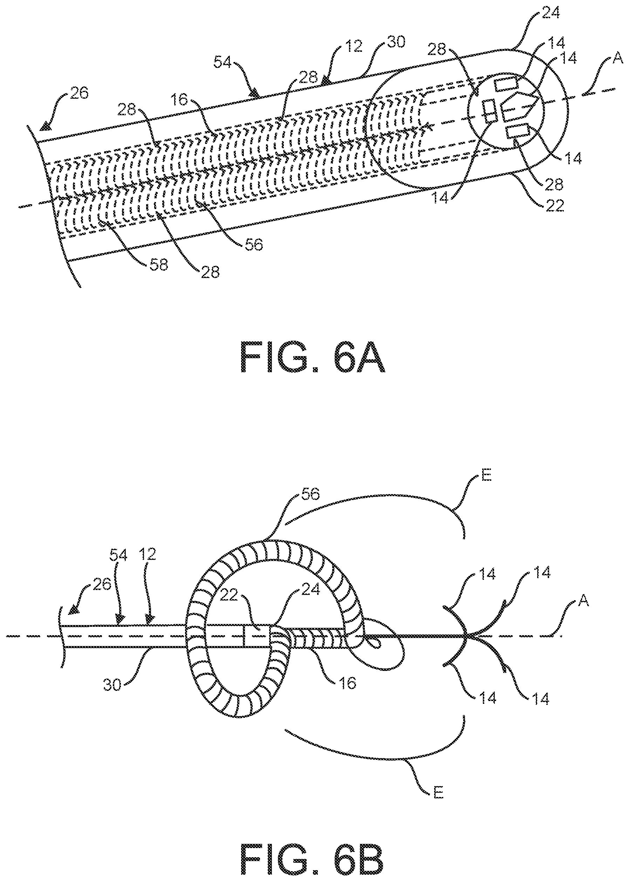

FIGS. 6A and 6B are schematic perspective views of a distal end 24 of a catheter for focal cardiac ablation by irreversible electroporation in undeployed and deployed configurations, respectively, according to some other embodiments of this disclosure. FIG. 6A shows a catheter 54 in the undeployed configuration. The catheter 54 is substantially identical to the catheter 10 described above, except that the return electrode 18 is replaced with a return electrode 56 and the electrical conductor 20 is replace with an electrical conductor 58. Unlike the embodiments describe above, the return electrode 56 is not disposed on the outer surface 30 of the catheter body 12.

The return electrode 56 is formed of an electrically conductive biocompatible material, such as stainless steel, platinum, platinum iridium alloy, platinum-clad tantalum, titanium, Nitinol, or a nickel-cobalt alloy, such as MP35N, for example. In some embodiments, the return electrode 56 is in the form of a wire coil. In some other embodiments, the return electrode 56 is in the form of a mesh or a hypo tube cut in a flexible pattern. The electrical conductor 58 is also formed of an electrically conductive material, such as stainless steel, platinum, platinum iridium alloy, platinum-clad tantalum, titanium, Nitinol, or a nickel-cobalt alloy, such as MP35N, for example. In some embodiments, the electrical conductor 58 is in the form of a wire coil. In some other embodiments, the electrical conductor 58 is in the form of a mesh or a hypo tube cut in a flexible pattern. In some embodiments, the return electrode 56 and the electrical conductor 58 may form a single, continuous wire coil. In embodiments in which the return electrode 56 and the flexible shaft 16 are both formed of different wire coils within the catheter body 12, the wire coil of the return electrode 56 may be a first coil and the wire coil of the flexible shaft 16 may be a second coil.

FIG. 6B shows the catheter 54 in the deployed configuration. As shown in FIG. 6B, the return electrode 56 is configured to deploy from the lumen 28 at the distal end 24. As with FIG. 58, the heart H and tissue T are omitted for clarity of illustration. The deployed return electrode 56 is configured to form a loop extending from the lumen 28. In some embodiments, articulation of the return electrode 56 may be controlled by extending and retracting the return electrode 56 from the lumen 28. In some embodiments, articulation of the return electrode 56 may be controlled by a pull wire (not shown). In some embodiments, the return electrode 56 is configured to selectively rotate around the longitudinal axis A. By rotating the return electrode 56 around the longitudinal axis A, the loop formed by the return electrode 56 moves relative to the plurality of tines 14, changing the position of the electrical field E. By extending and retracting the return electrode 56 from the lumen 28, the size of the loop may also be changed. In this way, the strength and position of the electrical field E may be tuned to provide more targeted ablation of the tissue T.

For clarity of illustration, all embodiments described above are shown with the plurality of tines 14 including four tines. However, it is understood that the disclosure encompasses embodiments with as few as two tines and as many as six tines.

As used herein, the phrase "within any range defined between any two of the foregoing values" literally means that any range may be selected from any two of the values listed prior to such phrase regardless of whether the values are in the lower part of the listing or in the higher part of the listing. For example, a pair of values may be selected from two lower values, two higher values, or a lower value and a higher value.

## Claims

1. A catheter (10) for focal cardiac ablation by irreversible electroporation, the catheter (10) comprising:
a flexible catheter body (12) extending from a proximal end (26) to a distal end (24), the catheter body (12) forming a lumen (28) extending from the proximal end (26) to the distal end (24), the catheter body (12) defining a longitudinal axis (A);
a plurality of tines (14) disposed at the distal end (24) of the catheter body (12), the plurality of tines (14) formed of an electrically conductive material and configured to deploy from the lumen (28) at the distal end (24) of the catheter body (12), each tine of the plurality of tines (14) configured to self-bias from a linear configuration within the lumen (28) to a curved configuration when deployed from the lumen (28);
a flexible shaft (16) formed of an electrically conductive material, the shaft (16) mechanically and electrically coupled to the plurality of tines (14) and extending through the lumen (28) from the proximal end (26) of the catheter body (12), the shaft (16) configured to deploy the tines from the lumen (28) when the shaft (16) is moved toward the distal end (24) of the catheter body (12); **characterised by**
a return electrode (18) disposed on an outer surface (30) of the catheter body (12); and
an electrical conductor (20) electrically coupled to the return electrode (18), the electrical conductor (20) extending through the catheter body (12) from the proximal end (26) of the catheter body (12).

2. The catheter (10) of claim 1, wherein the return electrode (18) includes a ring extending around a circumference of the catheter body (12).

3. The catheter (34) of claim 1, wherein the return electrode (18) includes a first ring (36) and a second ring (38), each of the first ring (36) and the second ring (38) extending around a circumference of the catheter body (12), the second ring (38) disposed proximally from the first ring (36).

4. The catheter (40) of claim 1, wherein the return electrode (18) includes at least one of: a wire coil (42), a wire mesh and a hypo tube cut in a flexible pattern, the return electrode (18) extending around a circumference of the catheter body (12).

5. The catheter (44) of claim 1, wherein catheter body (12) includes a deployable array (46) at the distal end (24) of the catheter body (12) and the return electrode (18) includes a plurality of return electrodes (50) disposed on the deployable array (46), the deployable array (46) including a plurality of splines (48), each spline of the plurality of splines (48) including at least one return electrode of the plurality of return electrodes (50).

6. The catheter (44) of claim 5, wherein the plurality of splines (48) are configured to transition between a retracted configuration in which each spline of the plurality of splines (48) is substantially parallel to the longitudinal axis (A) and a deployed configuration in which each spline of the plurality of splines (48) is bowed radially outward from the longitudinal axis (A).

7. The catheter (10) of any of claims 1-6, wherein the flexible shaft (16) is formed of a wire coil.

8. The catheter (10) of any of claim 1-7, wherein the return electrode (18) is disposed between 1 mm and 10 mm from the distal end (24) of the catheter body (12).

9. The catheter (10) of any of claim 1-8, wherein each tine of the plurality of tines (14) forms a curve extending from the distal end (24) of the catheter body (12) and away from the longitudinal axis (A) in the curved configuration.

10. A catheter (54) for focal cardiac ablation by irreversible electroporation, the catheter (54) comprising:
a flexible catheter body (12) extending from a proximal end (26) to a distal end (24) , the catheter body (12) forming a lumen (28) extending from the proximal end (26) to the distal end (24), the catheter body (12) defining a longitudinal axis (A);
a plurality of tines (14) disposed at the distal (24) end of the catheter body (12), the plurality of tines (14) formed of an electrically conductive material and configured to deploy from the lumen (28) at the distal end (24) of the catheter body (12), each tine of the plurality of tines (14) configured to self-bias from a linear configuration within the lumen (28) to a curved configuration when deployed from the lumen (28);
a flexible shaft (16) formed of an electrically conductive material, the shaft (16) mechanically and electrically coupled to the plurality of tines (14) and extending through the lumen (28) from the proximal end (26) of the catheter body (12), the shaft (16) configured to deploy the tines from the lumen (28) when the shaft (16) is moved toward the distal end (24) of the catheter body (12);
a return electrode (56) configured to deploy from the catheter body (12) at the distal end (24) of the catheter body (12); and
an electrical conductor (58) electrically coupled to the return electrode (56), the electrical conductor (58) extending through the catheter body (12) from the proximal end (26) of the catheter body (12), **characterised in that** said return electrode is configured to form a loop extending from the catheter body.

11. The catheter (54) of claim 10, wherein the return electrode (56) includes a first wire coil.

12. The catheter (54) of claim 11, wherein the electrical conductor (58) and the return electrode (56) each include the first wire coil.

13. The catheter (54) of any of claims 10-12, wherein the flexible shaft (16) is formed of a second wire coil.

14. The catheter (54) of any of claims 10-13, wherein each tine of the plurality of tines (14) forms a curve extending from the distal end (24) of the catheter body (12) and away from the longitudinal axis (A) in the curved configuration.

15. The catheter (54) of any of claims 10-14, wherein the loop formed by the return electrode (56) is configured to selectively rotate around the longitudinal axis (A).

## Patentansprüche

1. Katheter (10) zur fokalen Herzablation durch irreversible Elektroporation, wobei der Katheter (10) umfasst:
einen flexiblen Katheterkörper (12), der sich von einem proximalen Ende (26) zu einem distalen Ende (24) erstreckt, wobei der Katheterkörper (12) ein Lumen (28) bildet, das sich von dem proximalen Ende (26) zu dem distalen Ende (24) erstreckt, wobei der Katheterkörper (12) eine Längsachse (A) definiert;
eine Vielzahl von Zinken (14), die am distalen Ende (24) des Katheterkörpers (12) angeordnet sind, wobei die Vielzahl von Zinken (14) aus einem elektrisch leitenden Material gebildet und eingerichtet ist, dass sie sich aus dem Lumen (28) am distalen Ende (24) des Katheterkörpers (12) entfalten, wobei jede Zinke der Vielzahl von Zinken (14) eingerichtet ist, sich selbst von einer linearen Konfiguration innerhalb des Lumens (28) zu einer gekrümmten Konfiguration vorzuspannen, wenn sie aus dem Lumen (28) entfaltet wird;
einen flexiblen Schaft (16), der aus einem elektrisch leitenden Material gebildet ist, wobei der Schaft (16) mechanisch und elektrisch mit der Vielzahl von Zinken (14) gekoppelt ist und sich vom proximalen Ende (26) des Katheterkörpers (12) durch das Lumen (28) erstreckt, wobei der Schaft (16) eingerichtet ist, die Zinken aus dem Lumen (28) zu entfalten, wenn der Schaft (16) in Richtung des distalen Endes (24) des Katheterkörpers (12) bewegt wird; **gekennzeichnet durch**
eine Gegenelektrode (18), die auf einer Außenfläche (30) des Katheterkörpers (12) angeordnet ist; und
einen elektrischen Leiter (20), der elektrisch mit der Gegenelektrode (18) gekoppelt ist, wobei sich der elektrische Leiter (20) vom proximalen Ende (26) des Katheterkörpers (12) aus durch den Katheterkörper (12) erstreckt.

2. Katheter (10) nach Anspruch 1, wobei die Gegenelektrode (18) einen Ring aufweist, der sich um den Umfang des Katheterkörpers (12) erstreckt.

3. Katheter (34) nach Anspruch 1, wobei die Gegenelektrode (18) einen ersten Ring (36) und einen zweiten Ring (38) umfasst, wobei sich sowohl der erste Ring (36) als auch der zweite Ring (38) um einen Umfang des Katheterkörpers (12) herum erstrecken und der zweite Ring (38) proximal vom ersten Ring (36) angeordnet ist.

4. Katheter (40) nach Anspruch 1, wobei die Gegenelektrode (18) mindestens eines umfasst von: einer Drahtspule (42), einem Drahtgeflecht und einem in einem flexiblen Muster geschnittenen Hypo-Schlauch, wobei sich die Gegenelektrode (18) um einen Umfang des Katheterkörpers (12) erstreckt.

5. Katheter (44) nach Anspruch 1, wobei der Katheterkörper (12) eine entfaltbare Anordnung (46) am distalen Ende (24) des Katheterkörpers (12) aufweist und die Gegenelektrode (18) eine Vielzahl von Gegenelektroden (50) aufweist, die auf der entfaltbaren Anordnung (46) angeordnet sind, wobei die entfaltbare Anordnung (46) eine Vielzahl von Profilen (48) aufweist, wobei jedes Profil der Vielzahl von Profilen (48) mindestens eine Gegenelektrode der Vielzahl von Gegenelektroden (50) aufweist.

6. Katheter (44) nach Anspruch 5, wobei die Vielzahl von Profilen (48) eingerichtet ist, zwischen einer zurückgezogenen Konfiguration, in der jedes Profil der Vielzahl von Profilen (48) im Wesentlichen parallel zur Längsachse (A) verläuft, und einer entfalteten Konfiguration, in der jedes Profil der Vielzahl von Profilen (48) von der Längsachse (A) radial nach außen gebogen ist, übergeht.

7. Katheter (10) nach einem der Ansprüche 1 bis 6, wobei der flexible Schaft (16) aus einer Drahtspule gebildet ist.

8. Katheter (10) nach einem der Ansprüche 1 bis 7, wobei die Gegenelektrode (18) zwischen 1 mm und 10 mm vom distalen Ende (24) des Katheterkörpers (12) angeordnet ist.

9. Katheter (10) nach einem der Ansprüche 1 bis 8, wobei jeder Zinken der Vielzahl von Zinken (14) eine Kurve bildet, die sich vom distalen Ende (24) des Katheterkörpers (12) und von der Längsachse (A) weg in der gekrümmten Konfiguration erstreckt.

10. Katheter (54) zur fokalen Herzablation durch irreversible Elektroporation, wobei der Katheter (54) umfasst:
einen flexiblen Katheterkörper (12), der sich von einem proximalen Ende (26) zu einem distalen Ende (24) erstreckt, wobei der Katheterkörper (12) ein Lumen (28) bildet, das sich von dem proximalen Ende (26) zu dem distalen Ende (24) erstreckt, wobei der Katheterkörper (12) eine Längsachse (A) definiert;
eine Vielzahl von Zinken (14), die am distalen Ende (24) des Katheterkörpers (12) angeordnet sind, wobei die Vielzahl von Zinken (14) aus einem elektrisch leitenden Material gebildet und eingerichtet ist, dass sie sich aus dem Lumen (28) am distalen Ende (24) des Katheterkörpers (12) entfalten, wobei jede Zinke der Vielzahl von Zinken (14) eingerichtet ist, sich selbst von einer linearen Konfiguration innerhalb des Lumens (28) zu einer gekrümmten Konfiguration vorzuspannen, wenn sie aus dem Lumen (28) entfaltet wird;
einen flexiblen Schaft (16), der aus einem elektrisch leitenden Material gebildet ist, wobei der Schaft (16) mechanisch und elektrisch mit der Vielzahl von Zinken (14) gekoppelt ist und sich vom proximalen Ende (26) des Katheterkörpers (12) durch das Lumen (28) erstreckt, wobei der Schaft (16) eingerichtet ist, die Zinken aus dem Lumen (28) zu entfalten, wenn der Schaft (16) in Richtung des distalen Endes (24) des Katheterkörpers (12) bewegt wird;
eine Gegenelektrode (56), die eingerichtet ist, sich am distalen Ende (24) des Katheterkörpers (12) vom Katheterkörper (12) zu entfalten; und
einen elektrischen Leiter (58), der elektrisch mit der Gegenelektrode (56) gekoppelt ist, wobei sich der elektrische Leiter (58) vom proximalen Ende (26) des Katheterkörpers (12) durch den Katheterkörper (12) erstreckt, **dadurch gekennzeichnet, dass** die Gegenelektrode eingerichtet ist, eine Schleife zu bilden, die sich vom Katheterkörper erstreckt.

11. Katheter (54) nach Anspruch 10, wobei die Gegenelektrode (56) eine erste Drahtspule enthält.

12. Katheter (54) nach Anspruch 11, wobei der elektrische Leiter (58) und die Gegenelektrode (56) jeweils die erste Drahtspule enthalten.

13. Katheter (54) nach einem der Ansprüche 10-12, wobei der flexible Schaft (16) aus einer zweiten Drahtspule gebildet ist.

14. Katheter (54) nach einem der Ansprüche 10-13, wobei jeder Zinken der Vielzahl von Zinken (14) eine Kurve bildet, die sich vom distalen Ende (24) des Katheterkörpers (12) und von der Längsachse (A) weg in der gekrümmten Konfiguration erstreckt.

15. Katheter (54) nach einem der Ansprüche 10 bis 14, wobei die von der Gegenelektrode (56) gebildete Schleife eingerichtet ist, sich selektiv um die Längsachse (A) zu drehen.

## Revendications

1. Cathéter (10) pour une ablation cardiaque focalisée par électroporation irréversible, le cathéter (10) comprenant :
un corps de cathéter souple (12) qui est étendu depuis une extrémité proximale (26) jusqu'à une extrémité distale (24), le corps de cathéter (12) formant une lumière (28) qui est étendue depuis l'extrémité proximale (26) jusqu'à l'extrémité distale (24), le corps de cathéter (12) définissant un axe longitudinal (A) ;
une pluralité de pattes (14) qui sont disposées au niveau de l'extrémité distale (24) du corps de cathéter (12), les pattes de la pluralité de pattes (14) étant formées à partir d'un matériau électriquement conducteur et étant configurées pour être déployées depuis la lumière (28) au niveau de l'extrémité distale (24) du corps de cathéter (12), chaque patte de la pluralité de pattes (14) étant configurée pour réaliser une auto-sollicitation par poussée depuis une configuration linéaire à l'intérieur de la lumière (28) jusqu'à une configuration courbe lorsqu'elle est déployée depuis la lumière (28) ; et
un arbre souple (16) qui est formé à partir d'un matériau électriquement conducteur, l'arbre (16) étant couplé mécaniquement et électriquement à la pluralité de pattes (14) et étant étendu au travers de la lumière (28) depuis l'extrémité proximale (26) du corps de cathéter (12), l'arbre (16) étant configuré pour déployer les pattes depuis la lumière (28) lorsque l'arbre (16) est déplacé en direction de l'extrémité distale (24) du corps de cathéter (12) ; **caractérisé par** :
une électrode de retour (18) qui est disposée sur une surface externe (30) du corps de cathéter (12) ; et
un conducteur électrique (20) qui est couplé électriquement à l'électrode de retour (18), le conducteur électrique (20) étant étendu au travers du corps de cathéter (12) depuis l'extrémité proximale (26) du corps de cathéter (12).

2. Cathéter (10) selon la revendication 1, dans lequel l'électrode de retour (18) inclut un anneau qui est étendu le long d'une circonférence du corps de cathéter (12).

3. Cathéter (34) selon la revendication 1, dans lequel l'électrode de retour (18) inclut un premier anneau (36) et un second anneau (38), chaque anneau parmi le premier anneau (36) et le second anneau (38) étant étendu le long d'une circonférence du corps de cathéter (12), le second anneau (38) étant disposé de façon proximale par rapport au premier anneau (36).

4. Cathéter (40) selon la revendication 1, dans lequel l'électrode de retour (18) inclut au moins un élément constitutif parmi : une bobine de fil (42), un maillage de fil et un tube hypodermique qui est découpé selon un motif souple, l'électrode de retour (18) étant étendue le long d'une circonférence du corps de cathéter (12).

5. Cathéter (44) selon la revendication 1, dans lequel le corps de cathéter (12) inclut un réseau pouvant être déployé (46) au niveau de l'extrémité distale (24) du corps de cathéter (12) et l'électrode de retour (18) inclut une pluralité d'électrodes de retour (50) qui sont disposées sur le réseau pouvant être déployé (46), le réseau pouvant être déployé (46) incluant une pluralité d'éléments à courbure (48), chaque élément à courbure de la pluralité d'éléments à courbure (48) incluant au moins une électrode de retour de la pluralité d'électrodes de retour (50).

6. Cathéter (44) selon la revendication 5, dans lequel les éléments à courbure de la pluralité d'éléments à courbure (48) sont configurés de manière à effectuer une transition entre une configuration rétractée dans laquelle chaque élément à courbure de la pluralité d'éléments à courbure (48) est sensiblement parallèle à l'axe longitudinal (A) et une configuration déployée dans laquelle chaque élément à courbure de la pluralité d'éléments à courbure (48) est arqué radialement vers l'extérieur par rapport à l'axe longitudinal (A).

7. Cathéter (10) selon l'une quelconque des revendications 1 à 6, dans lequel l'arbre souple (16) est formé à partir d'une bobine de fil.

8. Cathéter (10) selon l'une quelconque des revendications 1 à 7, dans lequel l'électrode de retour (18) est disposée à une distance de l'extrémité distale (24) du corps de cathéter (12) qui est comprise entre 1 mm et 10 mm.

9. Cathéter (10) selon l'une quelconque des revendications 1 à 8, dans lequel chaque patte de la pluralité de pattes (14) forme une courbe qui est étendue depuis l'extrémité distale (24) du corps de cathéter (12) et à distance de l'axe longitudinal (A) dans la configuration courbe.

10. Cathéter (54) pour une ablation cardiaque focalisée par électroporation irréversible, le cathéter (54) comprenant :
un corps de cathéter souple (12) qui est étendu depuis une extrémité proximale (26) jusqu'à une extrémité distale (24), le corps de cathéter (12) formant une lumière (28) qui est étendue depuis l'extrémité proximale (26) jusqu'à l'extrémité distale (24), le corps de cathéter (12) définissant un axe longitudinal (A) ;
une pluralité de pattes (14) qui sont disposées au niveau de l'extrémité distale (24) du corps de cathéter (12), les pattes de la pluralité de pattes (14) étant formées à partir d'un matériau électriquement conducteur et étant configurées pour être déployées depuis la lumière (28) au niveau de l'extrémité distale (24) du corps de cathéter (12), chaque patte de la pluralité de pattes (14) étant configurée pour réaliser une auto-sollicitation par poussée depuis une configuration linéaire à l'intérieur de la lumière (28) jusqu'à une configuration courbe lorsqu'elle est déployée depuis la lumière (28) ;
un arbre souple (16) qui est formé à partir d'un matériau électriquement conducteur, l'arbre (16) étant couplé mécaniquement et électriquement à la pluralité de pattes (14) et étant étendu au travers de la lumière (28) depuis l'extrémité proximale (26) du corps de cathéter (12), l'arbre (16) étant configuré pour déployer les pattes depuis la lumière (28) lorsque l'arbre (16) est déplacé en direction de l'extrémité distale (24) du corps de cathéter (12) ;
une électrode de retour (56) qui est configurée pour être déployée depuis le corps de cathéter (12) au niveau de l'extrémité distale (24) du corps de cathéter (12) ; et
un conducteur électrique (58) qui est couplé électriquement à l'électrode de retour (56), le conducteur électrique (58) étant étendu au travers du corps de cathéter (12) depuis l'extrémité proximale (26) du corps de cathéter (12) ; **caractérisé en ce que** ladite électrode de retour est configurée de manière à former une boucle qui est étendue depuis le corps de cathéter.

11. Cathéter (54) selon la revendication 10, dans lequel l'électrode de retour (56) inclut une première bobine de fil.

12. Cathéter (54) selon la revendication 11, dans lequel le conducteur électrique (58) et l'électrode de retour (56) incluent chacun la première bobine de fil.

13. Cathéter (54) selon l'une quelconque des revendications 10 à 12, dans lequel l'arbre souple (16) est formé à partir d'une seconde bobine de fil.

14. Cathéter (54) selon l'une quelconque des revendications 10 à 13, dans lequel chaque patte de la pluralité de pattes (14) forme une courbe qui est étendue depuis l'extrémité distale (24) du corps de cathéter (12) et à distance de l'axe longitudinal (A) dans la configuration courbe.

15. Cathéter (54) selon l'une quelconque des revendications 10 à 14, dans lequel la boucle qui est formée par l'électrode de retour (56) est configurée pour tourner de façon sélective autour de l'axe longitudinal (A).
